# DEMANDE DE BREVET EUROPEEN

(11) **EP 0 595 703 A1**
(43) Date de publication de la demande: **04.05.1994**
(21) Numéro de dépôt: 93402611.3
(22) Date de dépôt: 25.10.1993
(51) Int. Cl.: C01B 33/34, C07D 323/00

(54) **Procédé de synthèse de zéolithes appartenant à la famille structurale de la faujasite**

(30) Priorité: 26.10.1992 FR 9212695
(71) Demandeur: SOCIETE NATIONALE ELF AQUITAINE, F-92400 Courbevoie (FR)
(72) Inventeur: Anglerot, Didier, F-64140 Lons (FR); Feron, Béatrice, F-64000 Pau (FR); Guth, Jean-Louis, F-68200 Brunstatt (FR)
(74) Mandataire: Boillot, Marc

(57) **Abrégé**

Procédé de synthèse de faujasite, présentant un rapport Si:Al supérieur à 1 et pouvant dépasser 3, en présence d'un agent structurant consistant en au moins un macrocycle carboné ayant 10 à 24 atomes par cycle et renfermant des hétéroatomes choisis parmi l'oxygène, l'azote, le soufre ou le silicium, le macrocycle étant introduit dans le milieu réactionnel sous forme de complexe sodique brut de synthèse.

## Description

L'invention a trait à un procédé de synthèse de zéolithes appartenant à la famille structurale de la faujasite.

Les zéolithes sont des tectosilicates cristallisés. Les structures sont constituées par des assemblages de tétraèdres TO₄ formant une charpente tridimensionnelle par la mise en commun des atomes d'oxygène. Dans les zéolithes du type aluminosilicate qui sont les plus communes, T représente le silicium tétravalent ainsi que l'aluminium trivalent. La charpente tridimensionnelle précitée présente des cavités et canaux qui possèdent des dimensions moléculaires et accueillent les cations compensant le déficit de charge lié à la présence de l'aluminium trivalent dans les tétraèdres TO₄, lesdits cations étant généralement échangeables.

D'une manière générale, la composition des zéolithes peut être représentée par la formule brute (M₂/ₙO; Y₂O₃ ; x ZO₂) à l'état déshydraté et calciné. Dans cette formule Z et Y désignent respectivement les éléments tétravalents et trivalents des tétraèdres TO₄, M représente un élément électropositif de valence n tel qu'un métal alcalin ou alcalino-terreux et constitue le cation de compensation échangeable, et x est un nombre pouvant varier de 2 à théoriquement l'infini auquel cas la zéolithe est une silice.

Chaque type de zéolithe possède une structure microporeuse distincte. La variation des dimensions et formes des micropores d'un type à l'autre, entraîne des changements dans les propriétés absorbantes. Seules les molécules ayant certaines dimensions et formes sont capables d'entrer dans les pores d'une zéolithe particulière. En raison de leurs caractéristiques remarquables les zéolithes conviennent tout particulièrement pour la purification ou la séparation de mélanges gazeux ou liquides comme, par exemple, la séparation d'hydrocarbures par adsorption sélective.

La composition chimique, avec en particulier la nature des éléments présents dans les tétraèdres TO₄ et la nature des cations de compensation échangeables, est également un facteur important intervenant dans la sélectivité d'adsorption et surtout dans les propriétés catalytiques de ces produits. Ils sont utilisés comme catalyseurs ou supports de catalyseurs dans le craquage, le reformage et en général la modification d'hydrocarbures ainsi que dans l'élaboration de nombreuses molécules.

De nombreuses zéolithes existent dans la nature : ce sont des aluminosilicates dont les disponibilités et propriétés ne répondent pas toujours aux exigences des applications industrielles. De ce fait, la recherche de produits ayant des propriétés nouvelles a conduit à la synthèse d'une grande variété de zéolithes essentiellement du type aluminosilicate. Parmi les nombreux exemples de ce type, on peut signaler la zéolithe A (US-A-2882243), la zéolithe X (US-A-2882244), la zéolithe Y (US-A-3130007), la zéolithe L (FR-A-1224154), la zéolithe T (FR-A-1223775), la zéolithe ZSM5 (US-A-3702886), la zéolithe ZSM12 (US-A-3832449 et la zéolithe ZSM4 (EP-A-0015132).

Les zéolithes de la famille structurale de la faujasite sont caractérisées par une structure de charpente tridimensionnelle qui peut être décrite à partir de l'assemblage de modules appelés cube-octaèdres. Chacun de ces modules est constitué de 24 tétraèdres contenant les éléments Si et Al et pontés par l'oxygène selon le principe décrit plus haut.

Dans le cube-octaèdre, les tétraèdres sont liés de manière à former huit cycles à six tétraèdres et six cycles à quatre tétraèdres. Chaque cube-octaèdre est relié en coordinence tétraédrique, à travers quatre cycles à six tétraèdres, à quatre cube-octaèdres voisins.

Il est commode, pour montrer les relations qui unissent les différents membres de la famille structurale, de considérer les plans structuraux dans lesquels les cube-octaèdres sont disposés aux sommets d'un réseau plan d'hexagones. Chaque cube-octaèdre est ainsi relié à trois voisins dans le plan structural.

La quatrième direction de liaison est dirigée alternativement de part et d'autre du plan structural et permet de relier les cube-octaèdres entre plans structuraux voisins et parallèles.

Selon les dispositions relatives de ces plans structuraux les uns par rapport aux autres, on peut obtenir:
- des séquences de trois plans structuraux distincts ABCABC... correspondant à une structure de symétrie cubique,
- des séquences de deux plans structuraux distincts ABAB... correspondant à une structure de symétrie hexagonale,
- des séquences plus complexes qui peuvent être régulières ou irrégulières.

Tous les solides appartenant à la famille structurale de la faujasite sont des polytypes et possèdent des canaux inter-connectés d'environ 0,8nm de diamètre. Ainsi la faujasite est une zéolithe naturelle dont la structure correspond à l'empilement de trois plans structuraux distincts ABC correspondant à une structure de symétrie cubique.

On peut obtenir, par synthèse à partir d'un gel d'aluminosilicate de sodium, des composés de même structure que la faujasite, lesdits composés étant appelés zéolithes X lorsque le rapport Si:Al du nombre d'atomes de silicium au nombre d'atomes d'aluminium est compris entre 1 et 1,5 et zéolithes Y lorsque ledit rapport Si:Al est supérieur à 1,5.

Le procédé général de synthèse des zéolithes de la famille structurale de la faujasite consiste en une cristallisation hydrothermale de gels d'aluminosilicates de compositions particulières renfermant un agent structurant, qui peut être un cation métallique et éventuellement un cation tel que le tétraéthylammonium.

Cette synthèse a ses limites. Elle ne permet d'obtenir ni les faujasites ayant des rapports Si:Al supérieurs à 3, ni des faujasites hexagonales pratiquement pures. En effet, les faujasites obtenues par ce procédé sont de structure cubique ou bien se présentent sous forme de mélanges de formes cubiques et hexagonales.

Le brevet français 2638444 d'ELF AQUITAINE décrit un procédé permettant d'obtenir par synthèse directe des faujasites dont le rapport Si:Al est supérieur à 3.

Ces faujasites à acidité plus élevée sont obtenues par un procédé mettant en oeuvre des macrocycles carbonés renfermant des hétéroatomes choisis parmi l'oxygène, l'azote ou le soufre.

L'utilisation de ces macrocycles permet d'orienter la synthèse vers l'obtention de faujasites cubiques, hexagonales ou un mélange des deux structures.

En effet, en utilisant comme structurant des macrocycles ayant 10 à 17 atomes par cycle, on obtient la faujasite cubique, tandis que les structurants renfermant 18 à 24 atomes dans leur cycle orientent la synthèse vers le polytype hexagonal.

Dans ce procédé, les macrocycles sont utilisés sous forme purifiée. Leur purification nécessite de nombreuses étapes de distillation et de séparation chromatographique.

Nous avons trouvé maintenant un procédé simplifié, donc plus économique, de préparation de faujasites de rapport Si:Al élevé.

Le procédé de synthèse de faujasites présentant un rapport Si:Al supérieur à 1 et pouvant dépasser 3, selon l'invention, consiste à réaliser un mélange réactionnel ayant un pH supérieur à 10 et renfermant de l'eau, une source de silicium tétravalent, une source d'aluminium trivalent, une source d'ions hydroxydes sous la forme d'une base forte minérale ou organique et un agent structurant consistant en au moins un macrocycle carboné ayant 10 à 24 atomes par cycle et renfermant des hétéroatomes choisis parmi l'oxygène, l'azote, le soufre ou le silicium, dans les proportions permettant la cristallisation en un composé de la famille structurale de la faujasite, maintenir le gel obtenu à une température au plus égale à 150°C et sous pression autogène pendant une durée suffisante pour effectuer la cristallisation du précurseur de la faujasite consistant en la faujasite emprisonnant l'agent structurant et à soumettre le précurseur à une calcination pour détruire l'agent structurant et produire la faujasite et il se caractérise en ce que le macrocycle carboné utilisé comme agent structurant est introduit dans le milieu réactionnel sous forme de complexe sodique brut de synthèse.

Avantageusement, la quantité d'agent structurant (ST) présente dans le mélange réactionnel est telle que le rapport molaire ST:Al^{III} aille de 0,1 à 4 et de préférence de 0,1 à 1. Les macrocycles renfermant entre 10 à 17 atomes conduisent à la faujasite cubique. La faujasite hexagonale est obtenue à l'aide de macrocycles renfermant au moins 18 atomes à condition que le rapport molaire Na₂O/Al₂O₃ soit ajusté entre 1,4 et 3,5 en fonciton du rapport SiO₂/Al₂O₃. Ainsi
pour SiO₂/Al₂O₃= 7 on prendra Na₂O/Al₂O₃ entre 1,4 et 2,4
pour SiO₂/Al₂O₃= 10 on prendra Na₂O/Al₂O₃ entre 1,8 et 2,8
pour SiO₂/Al₂O₃= 15 on prendra Na₂O/Al₂O₃ entre 2,2 et 3,5

Le complexe sodique du macrocycle carboné peut être synthétisé par tous procédés connus.

Les macrocycles les plus couramment utilisés sont des éthers couronnes. La synthèse des éthers couronnes met en général en oeuvre les éthers de glycol, selon la réaction générale: 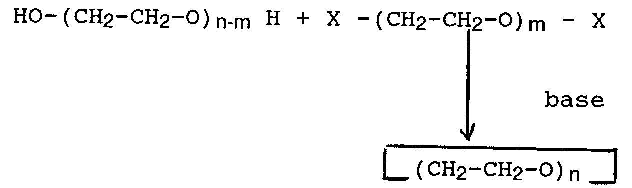
où X est généralement choisi parmi les groupes OH, tosylate ou halogénures et où n va de 4 à 8 et m de 0 à 4. Si X = OH, la cyclisation nécessite la présence d'un réactif comme le chlorure de tosyl.

La cyclo-oligomérisation de l'oxyde d'éthylène peut également être envisagée.

Pour la synthèse du 1,4,7,10,13,16- hexa-oxacyclo-octadécane (éther couronne "18 crown 6") on utilise en général les dérivés du triéthylène glycol.

Un procédé simple consiste en la cyclisation du triéthylène glycol en présence de chlorure de para-toluène sulfonyl et de la soude. L'éther couronne 18 crown 6 est obtenu sous forme de tosylate de son complexe sodique.

A la place de deux molécules de triéthylène glycol, il est possible d'utiliser une molécule d'hexaéthylène glycol.

Le même produit peut être obtenu par cyclisation du triéthylène glycol et du ditosylate de triéthylène glycol en présence de soude. Bien entendu, d'autres couples de diols et de ditosylates peuvent être utilisés, notamment le diéthylène glycol et le ditosylate de tétraéthylène glycol.

A la place des tosylates, il est possible d'utiliser d'autres groupes partants, notamment les halogènures, particulièrement les chlorures.

Ainsi le 18 crown 6 peut être obtenu, par exemple, à partir de triéthylène glycol et le dichlorure correspondant en présence de soude.

Le complexe sodique, brut de synthèse, de l'éther couronne 18 crown 6 obtenu après évaporation du solvant, se présente sous forme d'une huile. Cette huile peut être utilisée directement dans la synthèse hydrothermale de la faujasite. Cependant, il peut être préférable de séparer les produits de départ non réagis et les produits secondaires de la réaction.

Parmi les sources de silicium tétravalent Si^{IV} utilisables dans la préparation du mélange réactionnel destiné à former le gel d'aluminosilicate, on peut citer les silices solides finement divisées sous la forme d'hydrogels, d'aérogels ou de suspensions colloïdales, les silicates hydrosolubles tels que les silicates alcalins comme le silicate de sodium, les esters siliciques hydrolysables tels que les orthosilicates de tétraalcoyles de formule Si (OR)₄ dans laquelle R désigne un alcoyle en C₁ à C₄ tel que méthyle et éthyle. La source de silicium est mise en oeuvre sous la forme d'une solution aqueuse vraie, cas des silicates hydrosolubles, ou bien d'une suspension aqueuse qui peut être colloïdale, cas des silices finement divisées.

Conviennent comme sources d'aluminium trivalent Al^{III} les sels d'aluminium tels que sulfate, nitrate, chlorure, fluorure, acétate, les oxydes et hydroxydes d'aluminium, les aluminates et notamment les aluminates alcalins tels que l'aluminate de sodium, les esters d'aluminium tels que les aluminiums trialcoxydes de formule Al (OR)₃ dans laquelle R désigne un radical alcoyle en C₁ à C₄ tel que méthyle, éthyle ou propyle.

La source d'ions hydroxydes est choisie parmi les bases fortes minérales, notamment hydroxydes des métaux alcalins du groupe IA de la Classification Périodique des Eléments et hydroxydes des métaux alcalino-terreux Ca, Sr et Ba, et les bases fortes organiques, notamment hydroxydes d'ammoniums quaternaires, la préférence allant aux bases minérales et notamment à la soude, NaOH.

Le mélange réactionnel destiné à former le gel d'aluminosilicate peut encore renfermer des cations Mⁿ⁺ d'au moins un métal M, de valence n, autre que les métaux dont les hydroxydes sont des bases fortes, en quantité globale telle que le rapport molaire Mⁿ⁺:Al^{III}soit au plus égal à 0,4 et de préférence au plus égal à 0,3. Lesdits cations Mⁿ⁺ sont introduits dans ledit mélange réactionnel sous la forme de sels tels que sulfates, nitrates, chlorures ou acétates ou bien encore sous la forme d'oxydes. Comme exemples de tels cations, on peut citer notamment Co⁺⁺, Cd⁺⁺, Mg⁺⁺ et Ag⁺.

Pour former le mélange réactionnel destiné à former le gel d'aluminosilicate, on introduit le structurant sous forme de complexe sodique brut de synthèse, dans l'eau. En cas de formation d'un précipité ou de démixion d'une phase organique, on peut séparer ce précipité ou cette phase de la phase aqueuse.

A la solution aqueuse de structurant, on ajoute, à la température ambiante et dans un ordre quelconque, une solution aqueuse basique renfermant une base forte, les cations Mⁿ⁺, s'ils sont utilisés, une solution aqueuse de la source d'aluminium trivalent et une solution aqueuse ou une suspension, colloïdale ou non, de la source de silicium tétravalent.

Il faut bien sûr tenir compte dans le calcul de la base forte à ajouter, de la teneur en base libre contenue dans la solution aqueuse du structurant.

On peut introduire des germes de cristaux de faujasite hexagonale ou de polytype dans ce mélange.

Le mélange réactionnel peut être soumis à un mûrissement, en général à la température ambiante, pendant environ 6 heures à 20 jours, de préférence de 6 heures à 6 jours. En général, un mûrissement d'environ 24 heures est suffisant.

La cristallisation du gel d'aluminosilicate s'effectue en chauffant le mélange réactionnel à une température au plus égale à 150° et de préférence allant de 90 à 120° et sous pression correspondant au moins à la pression autogène du mélange réactionnel formant le gel. La durée de chauffage nécessaire à la cristallisation dépend du gel et de la température de cristallisation. Elle se situe généralement entre deux heures et quinze jours.

Ces cristaux obtenus, sont des cristaux du précurseur de la zéolithe, emprisonnant l'agent structurant dans ses pores et cavités. Après séparation du milieu réactionnel par filtration, les cristaux sont lavés à l'eau distillée jusqu'à obtention des eaux de lavage dont le pH est inférieur à 9. Les cristaux lavés sont ensuite séchés en étuve à une température comprise entre 50° et 100°C et de préférence aux environs de 70°C.

La zéolithe est obtenue à partir des cristaux du précurseur par calcination à une température supérieure à 300°C et de préférence comprise entre 400° et 700°C pendant une durée suffisante pour éliminer l'agent structurant.

Les zéolithes préparées par le procédé selon l'invention possèdent des rapports Si:Al supérieurs à 1 et pouvant dépasser 3. Elles sont de type faujasite et présentent une structure de symétrie cubique ou hexagonale.

Les faujasites obtenues par le procédé selon l'invention conviennent comme adsorbants sélectifs de molécules dont les dimensions sont inférieures à 0,8nm, ou encore, après avoir été soumises à des réactions d'échange avec des cations divers, comme catalyseurs ou composantes de catalyseurs utilisables dans les réactions de conversion catalytique de composés organiques et notamment des composés hydrocarbonés.

Les exemples suivant illustrent l'invention sans toutefois la limiter:

### EXEMPLES :

### EXEMPLE 1

Synthèse du complexe 18C6-NaOTS à partir du ditosylate de TEG et TEG
a) Synthèse du ditosylate de triéthylène glycol (TEG)
   Dans un ballon de 4 1 agité avec une tige de verre munie d'une ancre, et refroidi par un mélange eau-glace, on place 80 g (2 moles) de soude dissoute dans 400 ml d'eau et 105,12 g (0,70 mole) de triéthylène glycol dissous dans 400 ml THF.
   On ajoute goutte à goutte par une ampoule d'addition une solution de 243 g de chlorure de p-toluène sulfonyl (1,3 mole) dans 400 ml THF (sur une période de 2 heures).
   On laisse agiter encore 2 heures après la fin de l'ajout, puis on verse 1 litre d'un mélange eau + glace, ce qui a pour effet de précipiter le ditosylate que l'on filtre et sèche. Le rendement du produit brut est de 83 %. Le spectre RMN est entièrement conforme à celui attendu.
b) Synthèse du complexe.
   15,017 g de TEG (0,1 mole) sont dissous dans une suspension de 8 g de NaOH (0,2 mole) broyé en poudre dans 900 ml THF.
   On ajoute ensuite le ditosylate préalablement synthétisé (0,1 mole - 45,8 g). Le mélange est porté à reflux sous agitation pendant 2 heures, refroidi, filtré, puis le solvant est évaporé.

### EXEMPLE 2

Synthèse de complexe 18-C-6-NaCl à partir du dichlorure de TEG et TEG

Dans un ballon de 3 litres équipé d'une tige d'agitation, d'un réfrigérant et d'une ampoule d'addition, on met 112,5g de triéthylène glycol (0,75 mole) et 600 ml de THF. On commence l'agitation et on ajoute 70 ml d'une solution aqueuse de soude (66,4g - 1,65 mole).

Après 15 mn d'agitation, une solution de 3,6 dioxa-1,8 dichlorooctane (140,3g - 0,75 mole) (= triéthylène glycol dichloré) dans 100 ml de THF est ajoutée d'un seul coup. Le mélange est chauffé à reflux et agité vigoureusement pendant 18 heures. La solution est refroidie et le THF évaporé sous pression réduite. Le slurry marron résultant est dilué par le dichlorométhane (500 ml) et filtré (on enlève ainsi le chlorure de sodium formé à côté du complexe 18 crown 6-NaCl) puis le solvant est évaporé.

### EXEMPLE 3

Synthèse du complexe 18-C-6-NaOTS à partir de TEG

Dans un ballon de 4 litres, surmonté d'un réfrigérant, d'une ampoule d'addition et d'une tige d'agitation, on met en suspension 35,5g (0,887 mole) de soude broyée en poudre dans 1 litre de THF.

D'autre part, on mélange 33,37g de triéthylène glycol (0,222 mole) avec 42,36g de chlorure de p-toluènesulfonyl (0,222 mole) dans 1 litre de THF. Ce mélange est placé dans l'ampoule d'addition et ajouté à la suspension à reflux en 5 heures. Le mélange brunâtre obtenu est refroidi et le précipité blanc formé (TsONa+NaCl) est filtré, puis le solvant évaporé (sur évaporateur rotatif).

### EXEMPLE 4

Synthèse de faujasite avec le complexe de l'exemple 3

On préparait tout d'abord un gel d'aluminosilicate en opérant comme suit dans un récipient de capacité appropriée, le contenu dudit récipient étant maintenu sous agitation pendant toute la durée de l'opération. Dans le récipient on introduisait 8,9 parties d'eau, puis 0,58 partie de soude NaOH et, après dissolution de la soude, 2,52 parties du produit de l'exemple 3.

On ajoutait alors au contenu du récipient 1 partie d'un aluminate de sodium renfermant 56% d'Al₂O₃ et 37% de Na₂O et chauffait légèrement le mélange réactionnel pour dissoudre complètement l'aluminate. Après retour à température ambiante, on introduisait alors dans le récipient 8,2 partie d'une suspension colloïdale de silice renfermant 40% de SiO₂ et 60% d'eau.

On obtenait ainsi un gel d'aluminosilicate dont la composition molaire, rapportée à une mole d'Al₂O₃, était la suivante :
10 SiO₂ ; 1 Al₂O₃ ; 2,4 Na₂O ; 1 St ; 140 H₂O.

A ce gel on ajoutait, avant le mûrissement 0,045 parties de germes consistant en une faujasite hexagonale préalablement synthétisée dont on a broyé les cristaux.

Le gel obtenu était soumis à un mûrissement à température ambiante pendant 24 heures dans un récipient fermé.

Le gel mûri était ensuite placé dans un autoclave et maintenu à 110°C dans ce dernier pendant 144 heures pour former un produit cristallisé. Les cristaux formés étaient séparés du milieu réactionnel par filtration, puis lavés à l'eau distillée jusqu'à faible basicité (pH inférieur à 9) des eaux de lavage et enfin séchés à environ 60°C dans une étuve.

Ils se présentent sous forme de plaquettes hexagonales d'environ 3µm. Les cristaux séchés étaient ensuite calcinés à 600°C pendant 4 heures afin d'éliminer les molécules de l'éther couronne utilisé comme agent structurant et d'obtenir la zéolithe.

Le spectre de diffraction X de cet échantillon est semblable à celui de l'EMT.

### EXEMPLE 5

Synthèse de faujasite avec le complexe de l'exemple 1

6,9g du produit de l'exemple 1 sont mis en solution dans 24,3g d'eau. Le précipité est séparé par filtration puis l'on ajoute 1,34g de soude, 2,7g d'aluminate de sodium et 22,5g de ludox au filtrat pour obtenir un gel dont la composition molaire est la suivante:
10 SiO₂; 1 Al₂O₃; 2,2 Na₂O; 1 St, 140H₂O

Après un mûrissement de 24 heures à température ambiante, le gel d'aluminosilicate est porté à 110°C pendant 6 jours pour former un produit cristallisé. Les cristaux formés sont séparés du milieu réactionnel par filtration, puis lavés à l'eau distillée jusqu'à faible basicité des eaux de lavage et enfin séchés à environ 60°C.

Le spectre de diffraction de ce produit est identique à celui de l'EMT.

### EXEMPLE 6

Synthèse de faujasite avec le complexe de l'exemple 2.

On dissout 1,77 parties du produit de l'exemple 2 dans 162g d'eau et on neutralise la solution dont le pH est basique avec de l'acide chlorhydrique concentré jusqu'à pH=7. Puis on ajoute 0,84 partie de soude, 1 partie d'aluminate de sodium (renfermant 56% Al₂O₃ et 37% de Na₂O) et 8,24 parties d'une suspension colloïdale de silice renfermant 40% de SiO₂ et 60% d'eau.

Le gel d'aluminosilicate ainsi préparé a la composition molaire suivante :
10 SiO₂, 1 Al₂O₃, 2,9 Na₂O, 1 St, 140 H₂O

Le gel mûri pendant 24 heures à température ambiante en présence de germes (0,044 parties) est porté à 115°C pendant 65 heures, après quoi les cristaux sont séparés par filtration, lavés et séchés.

Le diffractogramme de cet échantillon est semblable à celui d'une faujasite cubique.

## Revendications

**1 -** Procédé de synthèse de faujasites présentant un rapport Si:Al supérieur à 1 et pouvant dépasser 3, consistant à réaliser un mélange réactionnel ayant un pH supérieur à 10 et renfermant de l'eau, une source de silicium tétravalent, une source d'aluminium trivalent, une source d'ions hydroxydes sous la forme d'une base forte, minérale ou organique, et un agent structurant consistant en au moins un macrocycle carboné ayant 10 à 24 atomes par cycle et renfermant des hétéro atomes choisis parmi l'oxygène, l'azote, le soufre ou le silicium, dans les proportions permettant la cristallisation en un composé de la famille structurale de la faujasite, maintenir le gel obtenu à une température au plus égale à 150°C et sous pression autogène pendant une durée suffisante pour effectuer la cristallisation du précurseur de la faujasite consistant en la faujasite emprisonnant l'agent structurant et à soumettre le précurseur à une calcination pour détruire l'agent structurant et produire la faujasite et se caractérisant en ce que le macrocycle carboné utilisé comme agent structurant est introduit dans le milieu réactionnel sous forme de complexe sodique brut de synthèse.

**2 -** Procédé selon la revendication 1 caractérisé en ce que la quantité d'agent structurant (st) présente dans le mélange est telle que le rapport molaire ST:Al^{III} aille de 0,1 à 4 et de préférence de 0,1 à 1.

**3 -** Procédé selon la revendication 1 ou 2 caractérisé en ce que l'agent structurant est un macrocycle renfermant 10 à 17 atomes.

**4 -** Procédé selon la revendication 1 ou 2 caractérisé en ce que l'agent structurant est un macrocycle renfermant au moins 18 atomes, que le rapport molaire Na₂O/Al₂O₃ est compris entre 1,4 et 3,5 et ajusté en fonction du rapport SiO₂/Al₂O₃.

**5 -** Procédé selon l'une des revendications 1 à 4 caractérisé en ce que le complexe sodique est un éther couronne.

**6 -** Procédé selon la revendication 5 caractérisé en ce que l'éther couronne renfermant entre 10 et 24 atomes est obtenu par la réaction de cyclisation. 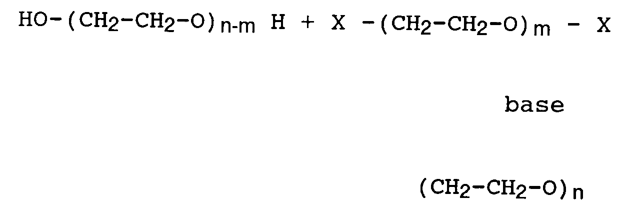 où X est choisi parmi les groupes OH, tosylate ou halogénure, où n va de 4 à 8 et m de 0 à 4.

**7 -** Procédé selon la revendication 5 caractérisé en ce que l'éther couronne est obtenu par cyclisation de l'oxyde d'éthylène.

**8 -** Procédé selon la revendication 5 caractérisé en ce que l'éther couronne est le 1,4,7,10,13,16 hexaoxacyclooctadécane (18 Crown 6).

**9 -** Procédé selon la revendication 8 caractérisé en ce que le 18 Crown 6 est obtenu par cyclisation du triéthylène glycol en présence de soude et de chlorure de p-toluène sulfonyl.

**10 -** Procédé selon la revendication 8 caractérisé en ce que le 18 Crown 6 est obtenu par cyclisation du triéthylène glycol et le triéthylène glycol ditosylate en présence de soude.
